# EUROPÄISCHE PATENTSCHRIFT

(11) **EP 1 605 828 B1**
(45) Veröffentlichungstag und Bekanntmachung des Hinweises auf die Patenterteilung: **16.05.2007**
(21) Anmeldenummer: 04722831.7
(22) Anmeldetag: 24.03.2004
(51) Int. Cl.: A61B 6/14

(54) **RÖNTGENGERÄT**
X-RAY DEVICE
APPAREIL DE RADIOGRAPHIE

(30) Priorität: 24.03.2003 DE 10313041; 06.10.2003 DE 10346288
(43) Veröffentlichungstag der Anmeldung: 21.12.2005
(73) Patentinhaber: Kaltenbach & Voigt GmbH, 88400 Biberach (DE)
(72) Erfinder: HACK, Alexander, 88400 Biberach (DE); ZELLER, Uwe, 88400 Biberach (DE); WEBER, Klaus, 88456 Grodt (DE); RICKERT, Martin, 88410 Bad Wurzach-Haidgau (DE)
(74) Vertreter: Schmidt-Evers, Jürgen
(86) Internationale Anmeldenummer: PCT/EP2004/003128
(87) Internationale Veröffentlichungsnummer: WO 2004/084732

(56) Entgegenhaltungen:
- WO-A-99/60928
- FR-A- 2 384 481
- FR-A- 2 614 491
- US-A- 2 955 205
- US-A- 3 767 931
- US-A- 5 553 115
- US-A- 5 708 696
- US-A1- 2002 122 537
- WEBBER R L ET AL: "Hand-held three-dimensional dental X-ray system: technical description and preliminary results." DENTO MAXILLO FACIAL RADIOLOGY. JUL 2002, Bd. 31, Nr. 4, Juli 2002 (2002-07), Seiten 240-248, XP002287006 ISSN: 0250-832X

## Beschreibung

Die vorliegende Erfindung betrifft ein Röntgengerät, insbesondere ein Röntgengerät welches in der zahnärztlichen Diagnostik verwendet wird.

Für eine umfassende und genaue Diagnostik in der Dentalmedizin ist der Einsatz zahnärztlicher Röntgengeräte unerlässlich. So können Fehlstellungen von Zähnen oder Beschädigungen der Zähne im Wurzelbereich lediglich anhand von zahnärztlichen Röntgenaufnahmen mit hoher Genauigkeit und Zuverlässigkeit diagnostiziert werden.

Die zum Einsatz kommenden Röntgentechniken können dabei im Wesentlichen in zwei unterschiedliche Kategorien unterteilt werden.

Bei einer sog. intraoralen Röntgenaufnahme wird ein röntgenstrahlungsempfindlicher Sensor, entweder ein Röntgenfilm oder ein Digitalsensor in Form eines in einem Gehäuse befindlichen Halbleitersensors in den Mundraum des Patienten eingebracht und von außen Röntgenstrahlung auf den zu untersuchenden Bereich gerichtet. Die in den Mundraum eingebrachten Sensoren weisen Abmessungen im Bereich von einigen Zentimetern auf und werden in erster Linie dazu verwendet, Röntgenbilder einzelner oder einiger weniger benachbarter Zähne zu erstellen.

Im Gegensatz zu der intraoralen Aufnahme, bei der der Röntgenstrahlungs-Sensor im Mundraum des Patienten angeordnet ist, befindet sich bei der sog. Panoramaaufnahme der Kopf des Patienten zwischen dem Röntgenstrahlungsgenerator und dem Sensor. Bei dieser Röntgentechnik wird der Röntgenkopf mit dem Strahlungsgenerator um den Kopf des Patienten herum geführt, währenddessen dauerhaft der Kieferbereich des Patienten von der Röntgenstrahlung, die wiederum von dem Röntgenstrahlungs-Detektor erfasst wird, durchleuchtet wird. Durch die besondere Bewegung des Röntgenkopfes wird eine sog. Panoramaaufnahme erstellt, welche eine Übersichtsdarstellung der Zähne des Unter- sowie des Oberkiefers des Patienten ist. Derartige Panoramaaufnahmen sind besonders gut dazu geeignet, Fehlstellungen einzelner Zähne zu erkennen.

Obwohl die Strahlenbelastung des Patienten sowohl bei der intraoralen Aufnahme als auch bei der Panoramaaufnahme im Vergleich zur Strahlenbelastung bei Röntgenuntersuchungen anderer Bereiche des Körpers verhältnismäßig gering ist, besteht auch in der zahnärztlichen Röntgendiagnostik die Verpflichtung, die Strahlenbelastung so gering wie möglich zu halten. Eine wesentliche Voraussetzung hierfür ist, dass unnötige Wiederholungen von fehlerhaften Röntgenaufnahmen, die auf eine falsche Positionierung des Sensors bzw. eine falsche Ausrichtung des Röntgenkopfes zurückzuführen sind, vermieden werden. Demzufolge besteht ein besonderes Bedürfnis dafür, die Ausrichtung und Anordnung des Röntgenkopfes bzw. des Sensorelementes zu optimieren.

Bei einer intraoralen Röntgenaufnahme besteht die Aufgabe darin, zum einen den Sensor innerhalb des Mundraumes in der gewünschten Position hinter dem zu untersuchenden Zahn anzuordnen, sowie zum anderen den Röntgenkopf zentral auf den Sensor auszurichten. Dies ist zum einen problematisch, da der Sensor bei geschlossenem Mund von außen nicht erkennbar ist, sowie zum anderen, da die Röntgenstrahlung selbst nicht sichtbar ist, so dass der Zahnarzt bzw. die die Röntgenaufnahme durchführende Person nicht sicher sein kann, ob die Strahlung letztendlich tatsächlich auf den gewünschten Bereich ausgerichtet ist. Bei der Durchführung einer Panoramaaufnahme besteht darüber hinaus die Schwierigkeit darin, dass der Röntgenkopf möglichst in einer bestimmten Ebene um den Kopf des Patienten herum geführt werden muss. Wird diese Ebene während des Umlaufs des Röntgenkopfs verlassen, so wird die Qualität der Panoramaaufnahme deutlich beeinträchtigt.

Um die Ausrichtung des Röntgenkopfes bei der Durchführung einer intraoralen Aufnahme zu erleichtern, ist es bislang bekannt, mechanische Hilfen zu verwenden. Beispielsweise ist es bekannt, den Halter für den Röntgenfilm bzw. für den digitalen Sensor so auszugestalten, dass ein Teil des Halters aus dem Mund des Patienten herausragt und anzeigt, an welcher Stelle sich der Sensor innerhalb des Mundraumes befindet. Hierdurch ist für den Zahnarzt zumindest erkennbar, auf welchen Bereich außerhalb des Kopfes die Röntgenstrahlung fallen sollte. Allerdings besteht nach wie vor das Problem, dass für den Zahnarzt nicht erkenntlich ist, ob die unsichtbare Röntgenstrahlung tatsächlich auf den gewünschten Bereich fällt.

Aus der US 5,553,115 ist ein Röntgengerät mit einer Bestrahlungsröhre bekannt, das eine optischen Einrichtung aufweist, mit der ein kreuzförmiges optisches Muster auf der Bestrahlungsfläche erzeugt werden kann. Die Bestrahlungsröhre weist ein Vorderende aus lichtdurchlässigem Material auf, das vom Ende des Hauptkörpers der Bestrahlungsröhre lösbar ist.

Der vorliegenden Erfindung liegt dementsprechend die Aufgabe zugrunde, eine Möglichkeit zu schaffen, die Ausrichtung des Röntgenkopfes bei der Durchführung einer zahnärztlichen Röntgenuntersuchung zu verbessern.

Die Aufgabe wird durch ein Röntgengerät, welches die Merkmale des Anspruchs 1 aufweist, gelöst.

Die erfindungsgemäße Lösung besteht darin, das Röntgengerät zusätzlich mit einer Zieleinrichtung zu versehen, deren optische Achse über eine optische Vorrichtung im Wesentlichen parallel zu der Röntgenstrahlung auf das zu untersuchende Objekt ausgerichtet ist. Die Zieleinrichtung kann dabei insbesondere eine - neben der Röntgenstrahlungsquelle - weitere Lichtquelle zum Erzeugen einer sichtbaren Pilotstrahlung umfassen, die mit Hilfe der optischen Vorrichtung parallel zu der Röntgenstrahlung auf das zu untersuchende Objekt ausgerichtet wird.

Mit Hilfe der Pilotstrahlung, bei der es sich beispielsweise um eine Laserstrahlung handeln kann, kann auf die Außenseite des zu untersuchenden Objekts eine optische Information aufgebracht werden, welche Rückschlüsse darauf zulässt, auf welchen Bereich die Röntgenstrahlung genau treffen wird. Bei der Durchführung einer intraoralen Aufnahme besteht somit die verbleibende Aufgabe für den Zahnarzt nur noch darin, festzulegen, auf welchen Bereich an der Außenseite des Patientenkopfes die Röntgenstrahlung auftreffen soll, damit der im Mundraum befindliche Sensor belichtet wird.

Die mit Hilfe der Pilotstrahlung erzeugte optische Information kann je nach Art der durchzuführenden Untersuchung unterschiedlicher Natur sein. Im Falle einer intraoralen Röntgenaufnahme ist es ausreichend, eine Information zu erzeugen, welche das Zentrum der auftreffenden Röntgenstrahlung kennzeichnet. Hierfür ist beispielsweise die Projizierung eines Punktes, eines Kreises - im Falle eines ringförmigen Pilotstrahles - oder eines Kreuzes ausreichend. Die Verwendung eines ringförmigen Pilotstrahles zur Erzeugung eines Kreises bietet dabei den Vorteil, dass hierdurch auch ein Rückschluss über die axiale Lage des Zieles im Vergleich zu dem Röntgenkopf erhalten werden kann, da bei einem nicht senkrechten Auftreffen der Pilotstrahlung der ringförmige Pilotstrahl zu einer Ellipse verzerrt werden würde.

Soll eine Panoramaaufnahme durchgeführt werden, so wird die Pilotstrahlung vorzugsweise derart ausgeführt, dass auf die Außenseite des Patientenkopfes eine horizontal ausgerichtete Linie projiziert wird. Die Verwendung einer Linie ist in diesem Fall von Vorteil, da hierdurch die Ebene gekennzeichnet werden kann, in der sich der Röntgenkopf um den Kopf des Patienten herum bewegen wird. Wird beispielsweise zuvor mit Hilfe eines Gesichtsbogens auf der Wange des Patienten angezeichnet, wie die Gebissebene verläuft, so kann mit Hilfe der Pilotstrahlung die Drehebene des Röntgenkopfes optimal auf die Gebissebene abgestimmt und damit die Panoramaaufnahme optimiert werden.

Eine weitere Möglichkeit besteht auch darin, durch die Verwendung mehrerer paralleler Linien als Pilotstrahlung eine Skala zu schaffen, welche die Möglichkeit eröffnet, den Abstand zwischen dem Röntgenkopf und dem Kopf des Patienten zu bestimmen.

Die Lichtquelle für die Pilotstrahlung, vorzugsweise ein Laser, der Licht im sichtbaren Bereich abgibt, also beispielsweise ein HeNe-Laser, oder eine Laserdiode ist üblicherweise ebenfalls in dem Röntgenkopf, der bereits die Röntgenquelle zum Erzeugen der Röntgenstrahlung enthält, angeordnet. Als optische Vorrichtung, welche die Laserstrahlung im Wesentlichen parallel zu der Röntgenstrahlung ausrichten soll, wird dann vorzugsweise ein im Strahlengang der Röntgenstrahlung angeordneter Strahlteiler verwendet, welcher für die Pilotstrahlung reflektierend wirkt, für die Röntgenstrahlung hingegen im wesentlichen durchlässig ist. Ein Material, welches diese erforderlichen Eigenschaften aufweist, ist beispielsweise Aluminium.

Gemäß einer vorteilhaften Weiterbildung der Erfindung ist die Lichtquelle für die Pilotstrahlung nicht dauerhaft eingeschaltet, sondern kann nur unter bestimmten Bedingungen aktiviert werden und wird nach der Durchführung einer Röntgenaufnahme zu bestimmten Zeitpunkten bzw. unter bestimmten Bedingungen abgeschalten. Wie später ausführlicher erläutert wird, ergeben sich hierdurch für den Zahnarzt oder eine andere die Röntgenaufnahme durchführende Person Möglichkeiten, auf einfache und schnelle Weise festzustellen, ob das Röntgengerät einsatzbereit ist, bzw. ob der Film richtig belichtet wurde oder ob sich der Patient bewegt hat.

Eine andere Weiterbildung der vorliegenden Erfindung befasst sich mit der Tatsache, dass bei der Durchführung von intraoralen Röntgenaufnahmen der Röntgenkopf unmittelbar auf die Außenseite des Patientenkopfes bzw. sehr nahe dazu angeordnet wird. Da die Pilotstrahlung für den Zahnarzt nur dann hilfreich ist, wenn sie für ihn auch gut sichtbar ist, ist zumindest das vordere Ende des Röntgentubus transparent ausgebildet. Hierdurch kann der Zahnarzt selbst für den Fall, dass er den Röntgenkopf unmittelbar auf den Patientenkopf aufsetzt, noch erkennen, auf welchen Bereich die Röntgenstrahlung letztendlich fallen wird.

Die optische Vorrichtung, also der Strahlteiler, kann im Übrigen auch dazu verwendet werden, ein optisches Beobachtungssystem auf die Achse des Röntgenstrahls auszurichten. Dies ist beispielsweise dann von Interesse, wenn es bei der Auswertung der Röntgenaufnahme von Nutzen ist, aus der Richtung des Röntgenstrahls auf das zu durchstrahlende Areal zu blicken, um dabei den Auftreffort und die laterale Ausdehnung des Bestrahlungsareals besser überwachen zu können. Beispielsweise kann hierzu eine Kamera oder eine andere Beobachtungseinrichtung verwendet werden, welche mit Hilfe des Strahlteilers auf das von der Röntgenstrahlung durchleuchtete Areal ausgerichtet wird.

Nachfolgend soll die Erfindung anhand der beiliegenden Zeichnungen näher erläutert werden. Es zeigen:
- Figur 1: ein erfindungsgemäßes zahnärztliches Röntgengerät in Gesamtdarstellung;
- Figur 2: eine schematische Darstellung der verschiedenen Lichtquellen innerhalb des Röntgenkopfes sowie des Verlaufs der Strahlungen;
- Figur 3: eine schematische Darstellung einer alternativen Möglichkeit für die Einkopplung der Pilotstrahlung;
- Figur 4: die durch die Pilotstrahlung gewährte Hilfestellung bei einer intraoralen Aufnahme;
- Figur 5: die durch die Pilotstrahlung gewährte Hilfestellung bei einer Panoramaaufnahme.

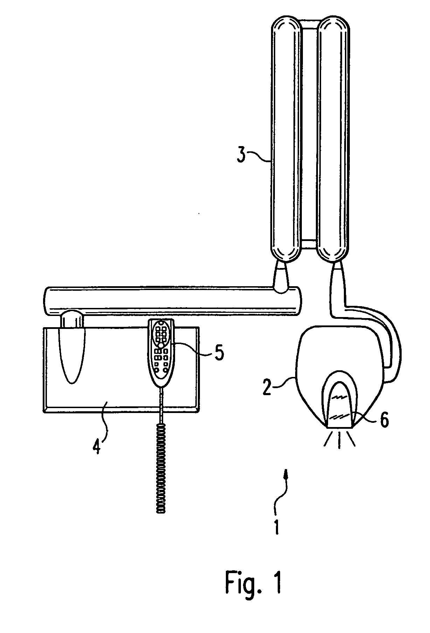

Wesentlicher Bestandteil des in Figur 1 dargestellten und allgemein mit dem Bezugszeichen 1 versehenen Röntgengeräts ist der die Röntgenlichtquelle enthaltene Röntgenkopf 2, der über ein Gestänge 3 einer Halterung bewegbar mit einer Zentraleinheit 4 des Röntgengerätes 1 verbunden ist. Die Halterung des Röntgenkopfes 2 ist derart, dass dieser in möglichst vielen Freiheitsgraden geschwenkt bzw. verschoben werden kann und somit vielseitig einsetzbar ist. Die dargestellte Halterung ist insbesondere für die Durchführung von intraoralen Röntgenaufnahmen vorgesehen, da bei dieser Aufnahmemethode der Röntgenkopf 2 während einer Aufnahme nicht bewegt wird. Bei den sogenannten Panoramaaufnahmen hingegen wird der Röntgenkopf 2 um eine bestimmte Achse verschwenkt, was automatisch erfolgen muss, um eine zufriedenstellende Bildqualität zu erhalten. Die hierfür erforderliche Anordnung zur geführten Bewegung des Röntgenkopfes 2 ist in Figur 1 nicht dargestellt.

Die Zentraleinheit 4 beinhaltet die wesentlichen Steuerelemente, welche zur Ansteuerung des Röntgenkopfes 2 bzw. der darin angeordneten Röritgenstrahlungsquelle erforderlich sind. Bestandteil der Zentraleinheit 4 ist ferner ein Eingabegerät 5, über welches die zur Durchführung der Röntgenuntersuchung wesentlichen Parameter (beispielsweise Strahlzeit, Strahldauer, Röhrenstrom (mA), Röhrenspannung (kV) oder dergleichen) eingegeben werden können. Anhand dieser Eingaben erzeugt die Zentraleinheit 4 Steuersignale, welche über Leitungen, die innerhalb des Gestänges 3 verlaufen, zu der in dem Röntgenkopf 2 angeordneten Röntgenstrahlungsquelle übermittelt werden. Die daraufhin erzeugte Röntgenstrahlung wird über einen Tubus 6 im vorderen Strahlungsaustrittsbereich des Röntgenkopfes 2 abgegeben. Wie später noch ausführlicher erläutert wird, ist der Röntgentubus 6 zumindest in seinem vorderen Bereich transparent ausgebildet, um dem Zahnarzt die Möglichkeit zu eröffnen, die durch die Pilotstrahlung gewährte Hilfestellung zu nutzen. Beispielsweise kann der Röntgentubus 6 aus Plexiglas gebildet sein.

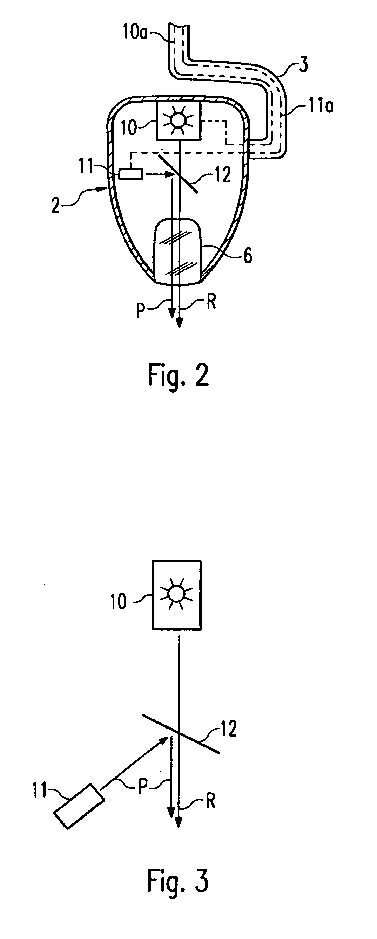

Figur 2 zeigt schematisch den inneren Aufbau des Röntgenkopfes 2, der zum einen die Röntgenstrahlungsquelle 10 zur Erzeugung der Röntgenstrahlung R sowie zum anderen die aus einer Pilotstrahlungs-Lichtquelle 11 sowie einem Strahlteiler 12 bestehende Zieleinrichtung aufweist, welche mit Hilfe eines Pilotstrahls P die Ausrichtung der Röntgenstrahlung R anzeigt. Sowohl die Röntgenstrahlungsquelle 10 als auch die Pilotstrahlungsquelle 11 werden von der in Fig. 1 dargestellten Zentraleinheit des Röntgengerätes angesteuert und sind hierzu mit dieser über zwei in dem Gestänge 3 verlaufende Leitungen 10a und 11a verbunden.

Da die Pilotstrahlung dem Zahnarzt eine optische Hilfestellung bieten soll, den Röntgenkopf 2 auf das zu untersuchende Objekt auszurichten, muss die Pilotstrahlung P sichtbar sein. Vorzugsweise wird hierfür eine Laserstrahlung aus dem roten Bereich verwendet. Beispielsweise könnten als Lichtquelle 11 für die Pilotstrahlung ein Helium-Neon- (HeNe)-Laser oder eine Laserdiode verwendet werden.

Das Ausrichten des Röntgenkopfes 2 mit Hilfe der Pilotstrahlung P wird umso mehr vereinfacht, je genauer die Röntgenstrahlung R und die Pilotstrahlung P zueinander ausgerichtet sind. Vorzugsweise sind beide Strahlungen koaxial zueinander ausgerichtet, eine gewisse Hilfestellung wird allerdings schon bereits dadurch erreicht, dass sie zumindest parallel zueinander oder lediglich mit einem geringen Winkel voneinander abweichen.

Die koaxiale Ausrichtung der Pilotstrahlung P zur Röntgenstrahlung R erfolgt über einen Strahlteiler 12, der im Strahlengang der Röntgenstrahlung R angeordnet ist. Wesentlich hierbei ist, dass der Strahlteiler 12 aus einem Material besteht, welches die Röntgenstrahlung R einfach zu durchdringen vermag, während hingegen der Pilotstrahl P reflektiert wird. Hierfür bietet sich beispielsweise an, den Strahlteiler 12 durch eine ebene Aluminiumplatte zu realisieren, da Aluminium die gewünschten Eigenschaften aufweist. Für den Fall, dass der Strahlteiler 12 vollständig eben ist, wird dann der von der Pilotstrahlungsquelle 11 emittierte Laserstrahl P punktförmig auf die Oberfläche des zu untersuchenden Projekts projiziert, wobei dieser Punkt dann das Zentrum der Röntgenstrahlung R darstellt.

Anzumerken ist, dass die Einbringung des Strahlteilers 12 in den Röntgenstrahlengang aufgrund der damit verbundenen sog. Eigenfilterung auch zum Aufhärten der Röntgenstrahlung genutzt werden kann, wobei die dabei entstehenden Streustrahlen noch im Inneren des Röntgenkopfes absorbiert werden. Hierbei ist von Vorteil, dass Aluminium ohnehin zur Eigenfilterung von Röntgenstrahlung verwendet wird, so dass bei einer entsprechenden Auslegung der Materialstärke der Spiegel bzw. Strahlteiler die Eigenfilterung bewirkt. Die Eigenfilterungseigenschaften und damit die technischen Daten des Geräts bleiben hierbei unverändert.

Figur 3 zeigt eine zu Figur 2 alternative Anordnung der Röntgenstrahlungsquelle 10, der Lichtquelle 11 für den Pilotstrahl P sowie des Strahlteilers 12, die sich darin unterscheidet, dass der Strahlteiler 12 nicht in einem 45 °-Winkel zu dem Verlauf der Röntgenstrahlung R angeordnet ist. Hinsichtlich der Funktionsweise des Strahlteilers 12 ergeben sich allerdings keine Unterschiede zur Anordnung in Figur 2.

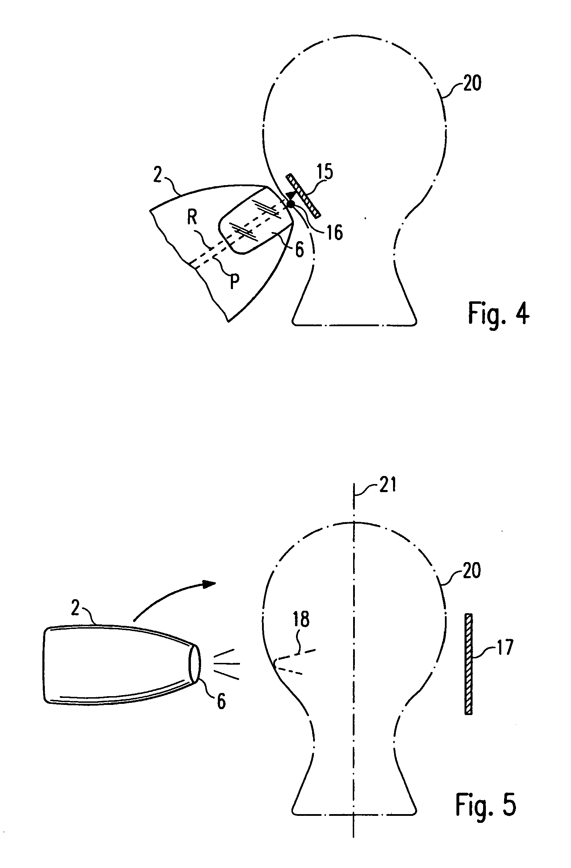

Die durch die Verwendung der Pilotstrahlung gewonnene Hilfestellung für den Zahnarzt bei der Ausrichtung des Röntgenkopfes soll nachfolgend anhand der Figuren 4 und 5 erläutert werden.

Figur 4 zeigt dabei den Anwendungsfall einer intraoralen Röntgenaufnahme, bei der ein röntgenstrahlungs-empfindliche Sensor 15 im Mundraum eines schematisch dargestellten Patientenkopfes 20 angeordnet ist. Bei dieser Untersuchung ist es erforderlich, den Röntgenkopf 2 derart auszurichten, dass die Röntgenstrahlung R möglichst optimal den Sensor 15 trifft.

Dies wird gemäß der vorliegenden Erfindung dadurch erleichtert, dass die Pilotstrahlung P an der Außenseite des Kopfes 20 eine Markierung 16 erzeugt, welche eindeutig anzeigt, in welchem Bereich bzw. an welcher Stelle die Röntgenstrahlung R auf den Kopf 20 des Patienten auftreffen wird. Für den Zahnarzt reduziert sich somit die Problematik ausschließlich darauf, die an der Außenseite des Patientenkopfes 20 erforderliche Stelle zu bestimmen, welche erforderlich ist, um den im Inneren des Mundraumes angeordneten Sensor 15 vollständig zu treffen. In diesem Fall ist besonders von Vorteil, dass zumindest der vordere Bereich des Tubus 6 des Röntgenkopfes 2 transparent ist, da selbst für den Fall, dass der Röntgenkopf 2 unmittelbar an die Außenseite des Patientenkopfes 20 angesetzt wird, noch die Stelle 16 erkennbar ist, auf welche der Pilotstrahl P auftrifft. Auch für den Fall, dass der Röntgentubus 6 gegenüber dem Gehäuse des Röntgenkopfes 2 hervorsteht, ist es von Vorteil, den Röntgentubus 6 transparent auszugestalten, um die Hilfestellung der Pilotstrahlung optimal nutzen zu können.

Gemäß einer Weiterbildung könnte vorgesehen sein, den transparenten Teil des Röntgentubus 6 abnehmbar zu gestalten, wobei dann die Möglichkeit besteht, eine Tubusverlängerung einzubauen. Auch bei dieser Variante ist vorgesehen, zumindest den vorderen Endbereich des Tubus transparent auszugestalten, beispielsweise könnte der transparente Aufsatz nach dem Einbau der Verlängerung wieder an das vordere Ende angesetzt werden, um die durch den Pilotstrahl zur Verfügung gestellte Hilfestellung nutzen zu können.

Eine zusätzliche Erleichterung der Ausrichtung des Röntgenkopfes 2 könnte ferner auch dadurch erreicht werden, dass der Halter für den Sensor 15 mit einer - nicht dargestellten - mechanischen Zielvorrichtung kombiniert wird, welche aufgrund der Tatsache, dass sie mit Hilfe einer Ausrichtungsvorrichtung definiert mit dem Halter für den Sensor 15 verbunden ist, im Bereich außerhalb des Patientenkopfes 20 eindeutig einen Bereich definiert, der von der Röntgenstrahlung R getroffen werden muss, um den Sensor 15 zu belichten. Wird diese Halterung zusätzlich mit einer Zielmarkierung ausgestattet, so kann eine korrekte Ausrichtung des Röntgenkopfes 2 beispielsweise dadurch angezeigt werden, dass der Pilotstrahl P die Zielmarkierung trifft. Die Qualität der hierdurch erhaltenen Röntgenaufnahme wird damit deutlich verbessert. Gleichzeitig wird sichergestellt, dass die Aufnahme nicht wegen einer falschen Ausrichtung des Röntgenkopfes 2 wiederholt werden muss. Die Zielmarkierung stellt in diesem Fall sozusagen eine auf den Sensor oder den Röntgenfilm verweisende "Zielscheibe" dar, über welche die korrekte Position bzw. Ausrichtung des Röntgenstrahls kontrolliert werden kann.

Figur 5 zeigt ein weiteres Anwendungsbeispiel der erfindungsgemäßen Zieleinrichtung. In diesem Fall wird eine sog. Panoramaaufnahme erstellt, bei der der Röntgenkopf 2 um die Zentralachse 21 des Patientenkopfes 20 geschwenkt wird. Gleichzeitig wird der auf der entgegengesetzten Seite des Patientenkopfes 20 liegende Sensor 17 in einer definierten Bewegung mitbewegt, wodurch insgesamt eine Übersichtsaufnahme der Zähne des Ober- sowie des Unterkiefers erhalten wird.

Eine Problematik bei dieser Aufnahmetechnik besteht darin, dass der Röntgenkopf 2 möglichst in der Gebissebene des Patienten bewegt werden soll, um hierdurch ein optimales Abbildungsergebnis zu erhalten. Die erfindungsgemäße Zieleinrichtung kann für diesen Fall derart ausgebildet werden, dass sie nicht einen einzelnen, koaxial zu der Röntgenstrahlung ausgerichteten Punkt an der Außenseite des Patientenkopfes 20 erzeugt, sondern stattdessen eine die Zentralachse der Röntgenstrahlung schneidende, horizontal ausgerichtete Linie 18. Die Linie 18 zeigt dabei an, in welcher Ebene der Röntgenkopf 2 umlaufen wird: Für den Zahnarzt wird somit die Aufgabe der Ausrichtung des Röntgenkopfes 2 darauf reduziert, die durch die Pilotstrahlung angezeigte Linie 18 in Einklang mit der Gebissebene des Patienten 20 zu bringen. Dies kann jedoch auf einfache Weise dadurch erfolgen, dass zuvor unter der Verwendung eines Gesichtsbogens auf der Wange des Patienten angezeichnet bzw. markiert wird, wie die Gebissebene verläuft. Der Zahnarzt legt in diesem Schritt den Verlauf bzw. die Anordnung der sog. "Frankfurter Horizontalen" fest. Der Röntgenkopf 2 muss letztendlich dann lediglich derart ausgerichtet werden, dass die von der Pilotstrahlung erzeugte Linie 18 die Markierungen trifft.

Das Erzeugen der Linie anstelle eines einzelnen Punktes mit Hilfe der Pilotstrahlung kann dabei in besonders einfacher Weise dadurch erfolgen, dass der in Figur 2 gezeigte Strahlteiler nicht eben ausgebildet sondern stattdessen leicht gewölbt ist. Hierdurch wird eine Auffächerung des Pilotstrahls erzielt, wodurch die gewünschte Linie erzeugt wird.

Anstelle der Projizierung eines einfachen Punktes oder einer Linie mit Hilfe des Pilotstrahls können allerdings auch andere Markierungen projiziert werden, welche eine geeignete Ausrichtung des Röntgenkopfes auf den Kopf des Patienten ermöglichen. Beispielsweise könnte auch ein ring- oder kreuzförmiger Pilotstrahl erzeugt werden, der koaxial zur Röntgenstrahlung verläuft. Eine andere Möglichkeit besteht ferner darin, mehrere parallel verlaufende Linien zu projizieren und hierdurch eine Skala zu schaffen, welche die Möglichkeit eröffnet, den Abstand zwischen der Röntgenlichtquelle und dem Patientenkopf zu bestimmen.

Abschließend soll noch darauf hingewiesen werden, dass die Pilotstrahlung neben einer Hilfestellung für die Ausrichtung des Röntgenkopfes ferner auch dazu verwendet werden kann, dem Zahnarzt einen Betriebszustand des Röntgengerätes anzuzeigen. Vorteilhafterweise ist der Pilotstrahl nämlich nicht dauerhaft aktiviert, sondern lediglich dann, wenn das vollständige Röntgensystem, also sowohl das Röntgengerät als auch eine eventuelle digitale Bilderfassungseinrichtung einsatzbereit sind. Üblicherweise erfolgt durch den Zahnarzt vor einer Aktivierung der Röntgenstrahlungsquelle noch eine kurze Kontrolle, ob das Aufnahmesystem auch einsatzbereit ist, da anderenfalls der Patient einer unnötigen Bestrahlung ausgesetzt werden würde. Eine entsprechende Kontrolle ist bei der vorteilhaften Weiterbildung der Erfindung überflüssig, da anhand des Vorhandenseins bzw. NichtVorhandenseins der Pilotstrahlung bei der Ausrichtung des Röntgenkopfes für den Zahnarzt automatisch ersichtlich ist, ob das gesamte System einsatzbereit ist. Um einen Fehler im Betrieb des Systems anzuzeigen, könnte die Pilotstrahlung beispielsweise auch moduliert werden, so dass die auf dem Gesicht des Patienten erscheinende Markierung blinkt.

Nach der Durchführung der Röntgenaufnahme wird der Filotstrahl manuell oder automatisch abgeschaltet, wobei er jedoch vorzugsweise für eine gewisse Nachläufzeit noch aktiviert bleibt. Diese sog. Nachlichtsteuerung bietet die Möglichkeit, dass der Zahnarzt nach der Aufnahme noch kontrollieren kann, ob der Patient zwischenzeitlich seinen Kopf bewegt hat bzw. sich die Ausrichtung des Röntgenkopfes auf den Pätientenkopf zwischenzeitlich verändert hat. Diese Nachlichtsteuerung ist insbesondere dann von Vorteil, wenn ein klassischer Röntgenstrahlungsdetektor, also ein Röntgenfilm verwendet wird, da der Zahnarzt noch vor einer Entwicklung des Filmes feststellen kann, ob die Aufnahme fehlerhaft war und somit der Vorgang wiederholt werden muss.

Der Strahlteiler kann im übrigen auch dazu verwendet werden, ein (nicht dargestelltes) optisches Beobachtungssystem auf die Achse des Röntgenstrahls auszurichten. Dies ist beispielsweise dann von Interesse, wenn es bei der Auswertung der Röntgenaufnahme von Nutzen ist, aus der Richtung des Röntgenstrahls auf das zu durchstrahlende Areal zu blicken, um dabei den Auftreffort und die laterale Ausdehnung des Bestrahlungsareals besser überwachen zu können. Beispielsweise kann hierzu eine Kamera oder eine andere Beobachtungseinrichtung verwendet werden, welche mit Hilfe des Strahlteilers auf das von der Röntgenstrahlung durchleuchtete Areal ausgerichtet wird.

Eine andere Möglichkeit, die insbesondere für die Erstellung von Panoramaaufnahmen von Nutzen ist, besteht darin, den Pilotstrahl aufzufächern und den Auftreffbereich der Pilotstrahlung gleichzeitig mit einer Beobachtungseinrichtung bzw. einer Kamera zu betrachten. Aus der Verzerrung der aufgefächerten Pilotstrahlung auf dem Gesicht des Patienten können dann der Abstand und die Ausrichtung des Patientenkopfes bestimmt und hieraus automatisch entsprechende Programmparameter für die Bewegung des Röntgenkopfes abgeleitet werden. Die Kombinationen der Pilotstrahlung mit einer optischen Beobachtungseinrichtung eröffnet somit die Möglichkeit, ein nahezu vollständig selbständig ablaufendes Aufnahmeverfahren zu realisieren. Die mit Hilfe der Kamera gewonnenen Informationen können im Übrigen auch dazu genutzt werden, Anweisungen zur Positionierung des Patientenkopfes abzuleiten.

Durch die vorliegende Erfindung wird somit eine Hilfestellung geschaffen, welche eine einfache und zuverlässige Ausrichtung des Röntgenkopfes eines Röntgengerätes auf eine gewünschte Position ermöglicht. Die hierzu erforderlichen Maßnahmen erfordern keine hohen Kosten und tragen zu einer deutlichen Steigerung der Aufnahmequalität bei. Die Verwendung des Pilotstrahl bietet darüber hinaus auch die Möglichkeit, einem Benutzer des Röntgengerätes auf einfache Weise anzuzeigen, ob das System einsatzbereit ist oder Systemfehler vorhanden sind, welche die Durchführung der Röntgenuntersuchung gefährden.

## Patentansprüche

1. Röntgengerät (1) mit einem Röntgentubus und mit einer Röntgenstrahlungsquelle (10) zum Erzeugen einer auf ein zu untersuchendes Objekt (20) zu richtenden Röntgenstrahlung (R), aufweisend eine optische Vorrichtung (12), über welche die optische Achse einer Zieleinrichtung im wesentlichen parallel zu der Röntgenstrahlung (R) auf das zu untersuchende Objekt (20) ausgerichtet ist,
wobei die Zieleinrichtung eine weitere Lichtquelle (11) zum Erzeugen einer sichtbaren Pilotstrahlung (P) umfaßt, welche über die optische Einrichtung auf das zu untersuchende Objekt (20) gerichtet ist,
wobei ein Austrittsbereich (6) für die Röntgenstrahlung (R) sowie die Pilotstrahlung (P) an dem die Röntgenstrahlungsquelle (10) enthaltenden Röntgenkopf (2) transparent ausgestaltet ist und den vorderen Endbereich eines Röntgentubus bildet, und
wobei der transparente Austrittsbereich (6) des Röntgentubus abnehmbar ist,
**dadurch gekennzeichnet,**
**dass** eine Tubusverlängerung vorgesehen ist, die nach Abnahme des Austrittsbereichs (6) in den Röntgentubus einsetzbar ist wobei der Austrittsbereich nach dem Einbau des Verlängerung wieder an das vordere Ende der Tubusverlängerung angesetzt werden kann.

2. Röntgengerät nach Anspruch 1,
**dadurch gekennzeichnet,**
**dass** die optische Einrichtung durch einen in dem Strahlengang der Röntgenstrahlung (R) angeordneten Strahlteiler (12) gebildet ist.

3. Röntgengerät nach Anspruch 2,
**dadurch gekennzeichnet,**
**dass** der Strahlteiler (12) für Licht aus dem sichtbaren Wellenlängenbereich im wesentlichen reflektierend wirkt und für die Röntgenstrahlung (R) im wesentlichen durchlässig ist.

4. Röntgengerät nach Ansprüche 3,
**dadurch gekennzeichnet,**
**dass** der Strahlteiler (12) aus Aluminium besteht.

5. Röntgengerät nach einem der Ansprüche 1 bis 4,
**dadurch gekennzeichnet,**
**dass** der Strahlteiler (12) die Funktion der Eigenfilterung miterfüllt.

6. Röntgengerät nach einem der vorherigen Ansprüche,
**dadurch gekennzeichnet,**
**dass** die Pilotstrahlung (P) im wesentlichen punktförmig als Lichtpunkt (16) auf das zu untersuchende Objekt projiziert wird, wobei die Röntgenstrahlung (R) und die Pilotstrahlung (P) koaxial zueinander ausgerichtet sind.

7. Röntgengerät nach einem der Ansprüche 1 bis 5,
**dadurch gekennzeichnet,**
**dass** die Pilotstrahlung (P) im wesentlichen als Linie (18) auf das zu untersuchende Objekt projiziert wird, wobei die Linie (18) die Zentralachse der Röntgenstrahlung (P) schneidet.

8. Röntgengerät nach Anspruch 7,
**dadurch gekennzeichnet,**
**dass** die Pilotstrahlung (P) als horizontal ausgerichtet Linie (18) abgebildet ist.

9. Röntgengerät nach Anspruch 7 oder 8,
**dadurch gekennzeichnet,**
**dass** die Linie (18) eine Umlaufebene für eine Umlaufbewegung des die Röntgenstrahlungsquelle (10) enthaltenden Röntgenkopfes (2) definiert.

10. Röntgengerät nach einem der Ansprüche 7 bis 9,
**dadurch gekennzeichnet,**
**dass** mehrere parallele Linien auf das zu untersuchende Objekt projiziert werden.

11. Röntgengerät nach einem der Ansprüche 2 bis 5 und einem der Ansprüche 7 bis 10,
**dadurch gekennzeichnet,**
**dass** der Strahlteiler (12) im Bereich des Auftreffpunkts der Pilotstrahlung (P) gewölbt bzw. gekrümmt ist.

12. Röntgengerät nach einem der Ansprüche 1 bis 5,
**dadurch gekennzeichnet,**
**dass** die Pilotstrahlung (P) im wesentlichen als eine kreis- oder kreuzförmige Markierung auf das zu untersuchende Objekt projiziert wird, wobei die Röntgenstrahlung (R) und die Pilotstrahlung (P) koaxial zueinander ausgerichtet sind.

13. Röntgengerät nach einem der vorherigen Ansprüche,
**gekennzeichnet durch**
einen Halter für einen zur Erfassung der Röntgenstrahlung (R) vorgesehenen und in dem Mundraum eines Patienten (20) anzuordnenden Sensors (15), wobei der Halter mit Hilfe einer Ausrichtungsvorrichtung definiert mit einer außerhalb des Mundraums angeordneten Zielvorrichtung verbunden ist, welche eine den Auftreffpunkt für die Pilotstrahlung (P) vorgebende Zielmarkierung aufweist.

14. Röntgengerät nach einem der vorherigen Ansprüche,
**dadurch gekennzeichnet,**
**dass** die Pilotstrahlung (P) rot ist.

15. Röntgengerät nach Anspruch 14,
**dadurch gekennzeichnet,**
**dass** die Lichtquelle für die Pilotstrahlung ein HeNe-Laser (12) oder ein Diodenlaser ist.

16. Röntgengerät nach einem der vorherigen Ansprüche,
**dadurch gekennzeichnet,**
**dass** der transparente Austrittsbereich (6) des Röntgenkopfes (2) aus Plexiglas besteht.

17. Röntgengerät nach einem der vorherigen Ansprüche,
**dadurch gekennzeichnet,**
**dass** die Pilotstrahlung (P) einen Betriebszustand des Röntgengeräts (1) und/oder einer Bilderfassungseinrichtung anzeigt.

18. Röntgengerät nach Anspruch 17,
**dadurch gekennzeichnet,**
**dass** die Pilotstrahlung (P) nur aktiviert ist, wenn das Röntgengerät (1) und/oder die Bilderfassungseinrichtung einsatzbereit sind.

19. Röntgengerät nach einem der vorherigen Ansprüche,
**dadurch gekennzeichnet,**
**dass** die Pilotstrahlung nach einer Aktivierung der Röntgenquelle für eine bestimmte Nachlaufzeit aktiviert bleibt.

20. Röntgengerät nach einem der vorherigen Ansprüche,
**gekennzeichnet durch**
ein optisches Beobachtungssystem zum Erfassen des Auftreffbereichs der Pilotstrahlung (P).

21. Röntgengerät nach Anspruch 20,
**dadurch gekennzeichnet,**
**dass** aus einer von dem optischen Beobachtungssystem erfaßten Verzerrung der Pilotstrahlung (P) auf der Oberfläche des zu untersuchenden Objekts Informationen hinsichtlich des Abstands und der Ausrichtung des zu untersuchenden Objekts gewonnen werden.

22. Röntgengerät nach Anspruch 21
**dadurch gekennzeichnet,**
**dass** anhand der Informationen hinsichtlich des Abstands und der Ausrichtung des zu untersuchenden Objekts Programmparameter für die Bewegung eines die Röntgenstrahlungsquelle (10) enthaltenden Röntgenkopfs (2) abgeleitet werden.

23. Röntgengerät nach einem der vorherigen Ansprüche,
**dadurch gekennzeichnet,**
**dass** die optische Einrichtung (12) ein optisches Beobachtungssystem auf die Achse des Röntgenstrahls ausrichtet.

24. Röntgengerät nach Anspruch 23,
**dadurch gekennzeichnet,**
**dass** es sich bei dem Beobachtungssystem um eine Kamera handelt.

25. Röntgengerät nach einem der vorherigen Ansprüche,
**dadurch gekennzeichnet,**
**dass** es sich um ein zahnärztliches Röntgengerät handelt.

## Claims

1. X-ray apparatus (1) with an X-ray tube and an X-ray radiation source (10) for generating X-ray radiation (R) to be directed at an object (20) to be investigated, having
an optical device (12) via which the optical axis of an aiming device is directed substantially parallel to the X-ray radiation (R) at the object to be investigated, the aiming device including a further light source (11) for the generation of visible pilot radiation (P) which is directed at the object to be investigated via the optical device,
an exit region (6) for the X-ray radiation (R) and the pilot radiation (P) on the X-ray head (2) containing the X-ray radiation source (10) being configured to be transparent and forming the forward end region of an X-ray tube, and the transparent exit region (6) of the X-ray tube being removable,
**characterized in that**,
a tube extension is provided which can be placed in the X-ray tube after removal of the exit region (6), wherein the exit region can, after installation of the extension, again be put in place on the forward end of the tube extension.

2. X-ray apparatus according to claim 1,
**characterized in that**,
the optical device is formed by means of a beam splitter (12) disposed in the beam path of the X-ray radiation (R).

3. X-ray apparatus according to claim 2,
**characterized in that**,
the beam splitter substantially reflects light in the visible wavelength range and is substantially transparent for the X-ray radiation (R).

4. X-ray apparatus according to claim 3,
**characterized in that**,
the beam splitter (12) is of aluminum.

5. X-ray apparatus according to any of claims 1 to 4,
**characterized in that**,
the beam splitter (12) also fulfils the function of self-filtering.

6. X-ray apparatus according to any preceding claim,
**characterized in that**,
the pilot radiation (P) is projected substantially in point form as a light point on the object to be investigated, the X-ray radiation (R) and the pilot radiation (P) being directed coaxially of one another.

7. X-ray apparatus according to any of claims 1 to 5,
**characterized in that**,
the pilot radiation (P) is projected substantially as a line (18) on the object to be investigated, the line (18) intersecting the central axis of the X-ray radiation (P).

8. X-ray apparatus according to claim 7,
**characterized in that**,
the pilot radiation (P) is imaged as a horizontally directed line (18).

9. X-ray apparatus according to claim 7 or 8,
**characterized in that**,
the line (18) defines a plane of rotation for a rotation movement of the X-ray head (2) containing the X-ray radiation source (10).

10. X-ray apparatus according to any of claims 7 to 9,
**characterized in that**,
a plurality of parallel lines are projected onto the object to be investigated.

11. X-ray apparatus according to any of claims 2 to 5 and any of claims 7 to 10,
**characterized in that**,
the beam splitter (12) is domed or curved in the region of the point of incidence of the pilot radiation (P).

12. X-ray apparatus according to any of claims 1 to 5,
**characterized in that**,
the pilot radiation (P) is projected onto the object to be investigated in substance as a circular or cross-shaped marking, the X-ray radiation (R) and the pilot radiation (P) being directed coaxially of one another.

13. X-ray apparatus according to any preceding claim,
**characterized by**
a holder for a sensor (15) provided for the detection of the X-ray radiation (R), to be arranged in the mouth of a patient,
the holder being connected in a defined manner, with the aid of an alignment device, with a targeting device disposed outside the mouth of the patient, which has a targeting marking indicating the point of incidence for the pilot radiation (P).

14. X-ray apparatus according to any preceding claim,
**characterized in that**,
the pilot radiation (P) is red.

15. X-ray apparatus according to claim 14,
**characterized in that**,
the light source for the pilot radiation is a HeNe laser (12) or a diode laser.

16. X-ray apparatus according to any preceding claim,
**characterized in that**,
the transparent exit region (6) of the X-ray head (2) is of Plexiglas.

17. X-ray apparatus according to any preceding claim,
**characterized in that**,
the pilot radiation (P) indicates an operational condition of the X-ray apparatus (1) and/or of an image detection device.

18. X-ray apparatus according to claim 17,
**characterized in that**,
the pilot radiation (P) is activated only when the X-ray apparatus (1) and/or the image detection device are ready for use.

19. X-ray apparatus according to any preceding claim,
**characterized in that**,
after an activation of the X-ray source the pilot radiation remains activated for a given overrun time.

20. X-ray apparatus according to any preceding claim,
**characterized by**
an optical observation system for detecting the region of incidence of the pilot radiation (P).

21. X-ray apparatus according to claim 20,
**characterized in that**,
information with regard to the spacing and the orientation of the object to be investigated is obtained based upon a distortion of the pilot radiation (P) on the surface of the object to be investigated that is detected by the optical observation system.

22. X-ray apparatus according to claim 21,
**characterized in that**,
on the basis of the information regarding the spacing and the orientation of the object to be investigated, program parameters for the movement of an X-ray head (2) containing the X-ray radiation source (10) are derived.

23. X-ray apparatus according to any preceding claim,
**characterized in that**,
the optical device (12) directs an optical observation system on the axis of the X-ray beam.

24. X-ray apparatus according to claim 23,
**characterized in that**,
the observation system is a camera.

25. X-ray apparatus according to any preceding claim,
**characterized in that**,
the X-ray apparatus is a dental X-ray apparatus.

## Revendications

1. Appareil de radiographie (1) avec un tube radiographique et une source de rayons X (10) pour produire un rayonnement X (R) à envoyer sur un objet (20) devant être examiné, présentant un dispositif optique (12) sur lequel l'axe optique d'un dispositif de visée est dirigé sur l'objet (20) à examiner pour l'essentiel parallèlement au rayonnement X (R),
le dispositif de visée comprenant une autre source lumineuse (11) pour produire un rayonnement pilote (P) visible qui est envoyé par le dispositif optique sur l'objet (20) à examiner,
une zone de sortie (6) pour le rayonnement X (R) ainsi que le rayonnement pilote (P) étant réalisée transparente sur la tête radiographique (2) contenant la source de rayons X (10) et formant la zone d'extrémité antérieure d'un tube radiographique, et la zone de sortie transparente (6) du tube radiographique (6) étant amovible,
**caractérisé en ce que**
un prolongement de tube est prévu qui, après dépose de la zone de sortie (6), peut être inséré dans le tube radiographique, la zone de sortie pouvant être remontée sur l'extrémité antérieure du prolongement de tube après le montage du prolongement.

2. Appareil de radiographie selon la revendication 1, **caractérisé en ce que** le dispositif optique est formé par un diviseur de rayons (12) placé sur le chemin de rayons du rayonnement X (R).

3. Appareil de radiographie selon la revendication 2, **caractérisé en ce que** le diviseur de rayons (12) est pour l'essentiel réfléchissant pour la lumière dans le domaine des longueurs d'onde visibles et pour l'essentiel transparent pour le rayonnement X (R).

4. Appareil de radiographie selon la revendication 3, **caractérisé en ce que** le diviseur de rayons (12) est constitué d'aluminium.

5. Appareil de radiographie selon l'une des revendications 1 à 4, **caractérisé en ce que** le diviseur de rayons (12) remplit également une fonction de filtrage propre.

6. Appareil de radiographie selon l'une des revendications précédentes, **caractérisé en ce que** le rayonnement pilote (P) est projeté pour l'essentiel ponctuellement sous forme de point lumineux (16) sur l'objet à examiner, le rayonnement X (R) et le rayonnement pilote (P) étant orientés coaxialement l'un à l'autre.

7. Appareil de radiographie selon l'une des revendications 1 à 5, **caractérisé en ce que** le rayonnement pilote (P) est projeté pour l'essentiel sous forme de ligne (18) sur l'objet à examiner, la ligne (18) coupant l'axe central du rayonnement X (R).

8. Appareil de radiographie selon la revendication 7, **caractérisé en ce que** le rayonnement pilote (P) est projeté sous la forme d'une ligne (18) orientée horizontalement.

9. Appareil de radiographie selon la revendication 7 ou 8. **caractérisé en ce que** la ligne (18) définit un plan de circulation pour un mouvement de circulation de la tête radiographique (2) contenant la source de rayons X (10).

10. Appareil de radiographie selon l'une des revendications 7 à 9, **caractérisé en ce que** plusieurs lignes parallèles sont projetées sur l'objet à examiner.

11. Appareil de radiographie selon l'une des revendications 2 à 5 et l'une des revendications 7 à 10, **caractérisé en ce que** le diviseur de rayons (12) est bombé ou incurvé au niveau du point d'incidence du rayonnement pilote (P).

12. Appareil de radiographie selon l'une des revendications 1 à 5, **caractérisé en ce que** le rayonnement pilote (P) est projeté pour l'essentiel sous la forme d'un repère circulaire ou cruciforme sur l'objet à examiner, le rayonnement X (R) et le rayonnement pilote (P) étant orientés coaxialement l'un à l'autre.

13. Appareil de radiographie selon l'une des revendications précédentes, **caractérisé par** un support pour un capteur (15) prévu pour détecter le rayonnement X (R) et devant être placé dans la bouche d'un patient (20), le support étant relié à l'aide d'un dispositif d'orientation de manière définie avec un dispositif de visée placé à l'extérieur de la bouche, qui présente un marquage cible définissant le point d'incidence du rayonnement pilote (P).

14. Appareil de radiographie selon l'une des revendications précédentes, **caractérisé en ce que** le rayonnement pilote (P) est rouge.

15. Appareil de radiographie selon la revendication 14, **caractérisé en ce que** la source lumineuse pour le rayonnement pilote est un laser HeNe (12) ou un laser à diode.

16. Appareil de radiographie selon l'une des revendications précédentes, **caractérisé en ce que** la zone de sortie transparente (6) de la tête radiographique (2) est en plexiglas.

17. Appareil de radiographie selon l'une des revendications précédentes, **caractérisé en ce que** le rayonnement pilote (P) affiche un état de service de l'appareil de radiographie (1) et/ou d'un dispositif de capture d'image.

18. Appareil de radiographie selon la revendication 17, **caractérisé en ce que** le rayonnement pilote (P) n'est activé que lorsque l'appareil de radiographie (1) et/ou le dispositif de capture d'image est prêt à fonctionner.

19. Appareil de radiographie selon l'une des revendications précédentes, **caractérisé en ce que** le rayonnement pilote reste activé pendant un certain temps après une activation de la source de rayons X.

20. Appareil de radiographie selon l'une des revendications précédentes, **caractérisé par** un système optique d'observation pour examiner la zone d'incidence du rayonnement pilote (P).

21. Appareil de radiographie selon la revendications 20, **caractérisé en ce qu'**à partir d'une déformation du rayonnement pilote (P) relevée par le système optique d'observation sur la surface de l'objet à examiner, on en déduit des informations sur la distance et l'orientation de l'objet à examiner.

22. Appareil de radiographie selon la revendication 21, **caractérisé en ce qu'**à l'aide des informations relatives à la distance et à l'orientation de l'objet à examiner, on en déduit des paramètres de programme pour le mouvement d'une tête radiographique (2) contenant la source de rayons X (10).

23. Appareil de radiographie selon l'une des revendications précédentes, **caractérisé en ce que** le dispositif optique (12) dirige un système d'observation optique sur l'axe du rayonnement X

24. Appareil de radiographie selon la revendication 23. **caractérisé en ce que** le système d'observation est une caméra.

25. Appareil de radiographie selon l'une des revendications précédentes, **caractérisé en ce qu'**il s'agit d'un appareil de radiographie dentaire.
